(19) Europäisches Patentamt / European Patent Office / Office européen des brevets

(11) **EP 2 857 014 B1**

(12) **EUROPEAN PATENT SPECIFICATION**

(45) Date of publication and mention
of the grant of the patent:
**22.06.2016 Bulletin 2016/25**

(51) Int Cl.:
*A61K 9/28* (2006.01)     *A61K 9/48* (2006.01)
*A61K 9/50* (2006.01)     *A61K 31/138* (2006.01)
*A61K 31/616* (2006.01)

(21) Application number: **14199791.6**

(22) Date of filing: **05.05.2011**

(54) **A pharmaceutical composition comprising aspirin and bisoprolol**

Pharmazeutische Zusammensetzung mit Aspirin und Bisoprolol

Composition pharmaceutique comprenant de l'aspirine et du bisoprolol

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **06.05.2010 US 331916 P**

(43) Date of publication of application:
**08.04.2015 Bulletin 2015/15**

(62) Document number(s) of the earlier application(s) in
accordance with Art. 76 EPC:
**11720589.8 / 2 566 472**

(73) Proprietor: **CAL INTERNATIONAL LIMITED
Dublin 6, (IE)**

(72) Inventors:
• **Donegan, Ann
County Tipperary (IE)**
• **Closs, Stephen Paul
Brooklin, Ontario (CA)**
• **Mallappa, Danashankar
Mississauga, Ontario L5N 8K5 (CA)**

(74) Representative: **O'Brien, John Augustine et al
John A. O'Brien & Associates
Third Floor,
Duncairn House,
14 Carysfort Avenue
Blackrock, Co Dublin (IE)**

(56) References cited:
**WO-A1-2008/095263**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Introduction

This invention relates to a pharmaceutical composition comprising aspirin and a beta blocker.

Statements of Invention

According to the invention there is provided a pharmaceutical capsule which contains only two pharmaceutically active ingredients, the capsule containing:-

a tablet which comprises aspirin as the first pharmaceutically active ingredient; and

granules surrounding the tablet, the granules comprising bisoprolol fumarate as the second pharmaceutically active ingredient,

the capsule containing from 50mg to 125mg of aspirin and from 1.25mg to 15mg of bisoprolol fumarate,

the aspirin tablet being coated with a barrier layer to prevent chemical interaction between the aspirin and the bisoprolol fumarate and to prevent interaction between aspirin and moisture in the capsule.

The aspirin is released from the capsule in vivo (biological fluids) to provide a peak concentration in advance of a peak concentration of bisoprolol.

The bisoprolol fumarate may be released immediately from the capsule to provide a peak concentration of bisoprolol within about 4 hours.

The aspirin may be also released immediately from the capsule to provide a peak concentration within about 2 hours.

The optimum effect may be achieved when both the bisoprolol and aspirin are released quickly to provide peak concentrations within a few hours.

The moisture barrier around the aspirin tablet may prevent degradation occurring. Absorption of moisture into the tablet from the capsule may be prevented and interaction between the bisoprolol fumarate and the aspirin may also prevent.

In one case, when the capsule is stored at 25°C and 60% relative humidity for 1 month, the concentration of any single impurity is not greater than 0.3% w/w.

In one case, when the capsule is stored at 25°C and 60% relative humidity for 3 months, or for 6 months, or for 12 months, or for up to 24 months, the concentration of any single impurity is not greater than 0.3% w/w.

In one case, when the capsule is stored at 25°C and 60% relative humidity for 1 month the concentration of any single impurity is not greater than 0.2% w/w.

In one case, when the capsule is stored at 25°C and 60% relative humidity for 3 months, or for 6 months, or for 12 months, or for up to 24 months, the concentration of any single impurity is not greater than 0.2% w/w.

The aspirin tablet may comprise from 75mg to 100mg of aspirin. The tablet may comprise approximately 75mg of aspirin or approximately 100mg of aspirin or approximately 86mg of aspirin. These are preferred to achieve the beneficial effects of aspirin, especially anti-platelet activity.

The capsule may contain any desired amount of bisoprolol from 1.25 to 10mg.

In one case the capsule contains approximately 5mg of bisoprolol fumarate.

In another case the capsule contains approximately 10mg of bisoprolol fumarate.

Other examples include 1.25mg or 3.75mg of bisoprolol fumarate.

These are preferred to achieve the beneficial effects of bisoprolol, especially β-blocking activity.

Brief Description of the Drawings

The invention will be more clearly understood from the following embodiments thereof given by way of example only with reference to the accompanying drawings, in which:-

Fig. 1 is a graph of in-vitro dissolution over time of an aspirin tablet used in a capsule of the invention;

Fig. 2 is a graph of in vivo absorption over time of aspirin from capsule of the invention in comparison with Hjertemagnyl 75mg film-coated tablets (Nycomed, Denmark);

Fig. 3 is a graph of in vivo levels over time of salicylic acid from capsule of the invention in comparison with Hjertemagnyl 75mg film-coated tablets (Nycomed, Denmark);

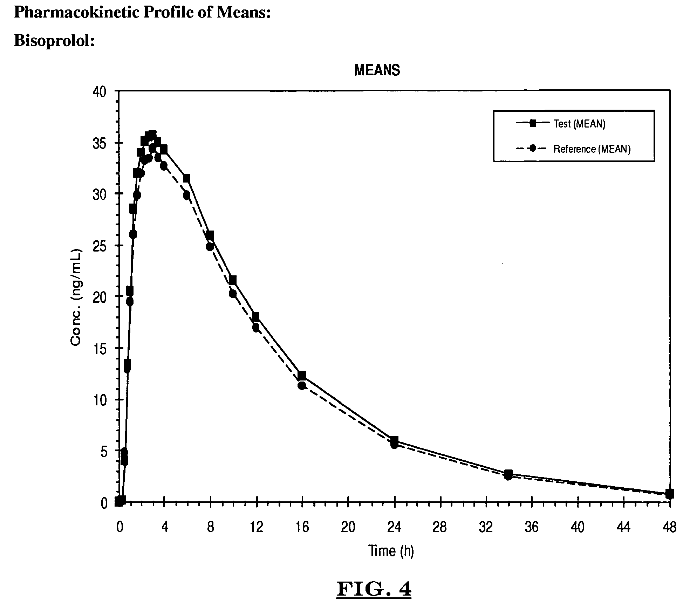

Fig. 4 is a graph of in vivo levels over time of bisoprolol from the capsule of the invention in comparison with Emcor (Merck);

Fig. 5 is a typical chromatogram from the analysis of aspirin in aspirin / bisoprolol capsules stored for 3 months at 25°C, without any coating on the aspirin tablet;

Fig. 6 is a typical chromatogram from the analysis of bisoprolol in aspirin / bisoprolol capsules stored for 3 months at 25°C, without any coating on the aspirin tablet;

Fig. 7 is a typical chromatogram from the analysis of aspirin in aspirin / bisoprolol capsules according to the invention, stored for 24 months at 25°C; and

Fig. 8 is a typical chromatogram from the analysis of bisoprolol in aspirin / bisoprolol capsules according to the invention, stored for 24 months at 25°C.

Detailed Description

A single dose capsule according to the invention was formulated as follows:-

A. Aspirin Tablet

The following amounts of aspirin and excipients and vehicles were mixed together and the mixture thus formed was compressed in a tablet press to form tablets which were approximately 5 mm in diameter, and weighed approximately 100 mg (90-130mg)

| Quantities for 1,000 tablets | |
| --- | --- |
| Aspirin | 75g |
| Starch | 9g |
| Microcrystalline cellulose | 6.6g |
| Stearic acid | 0.4g |

The aspirin is processed with starch and blended with microcrystalline cellulose followed by the magnesium stearate. Tablets are then formed using a tabletting machine by conventional techniques (Pharmaceutical Dosage Forms Tablets Vol 3 Edited by Herbert Lieberman, Leon Lachman, Joseph Schwartz).

B. Coating Material

The coating material used was Opadry AMB™ from Colorcon. The aspirin tablet of A was coated to 5% weight gain with this coating material by conventional techniques (Pharmaceutical Dosage Forms Tablets Vol 3 Edited by Herbert Lieberman, Leon Lachman, Joseph Schwartz).

C. Bisoprolol Fumarate

5 mg of bisoprolol fumarate available from Unichem was blended with inert excipients such as microcrystalline cellulose and magnesium stearate, 155mg used for each capsule. The particle size of bisoprolol fumarate is controlled to 90% < 425μm.

D. Capsule

The capsule is of a conventional capsule material such as hard gelatine material size 0 or 1.

Combination Product

A 75 mg aspirin tablet from A coated with the coating material of B was placed in the capsule of D and surrounded by the bisoprolol fumarate of C.

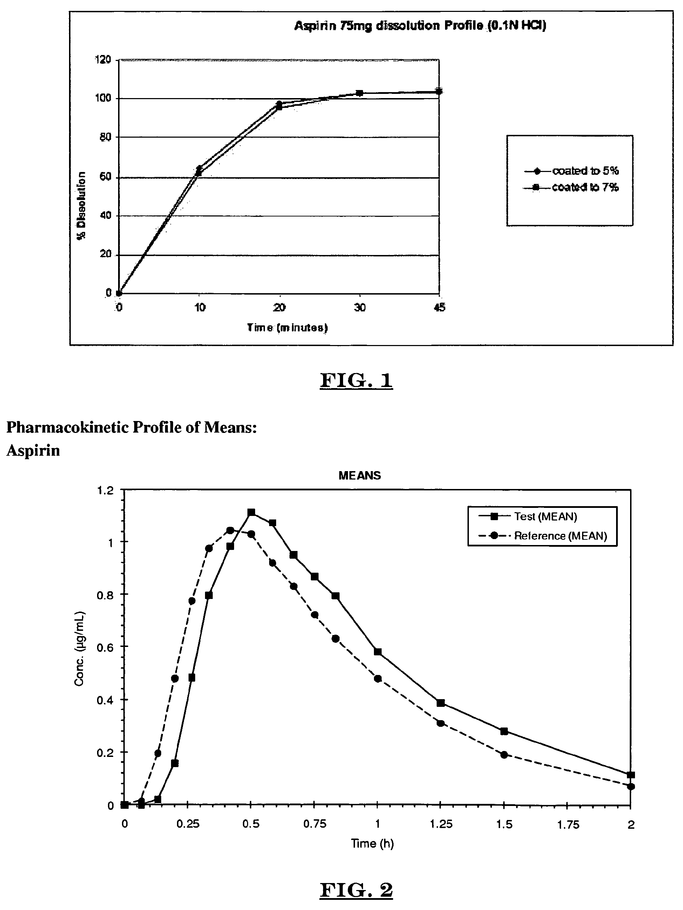

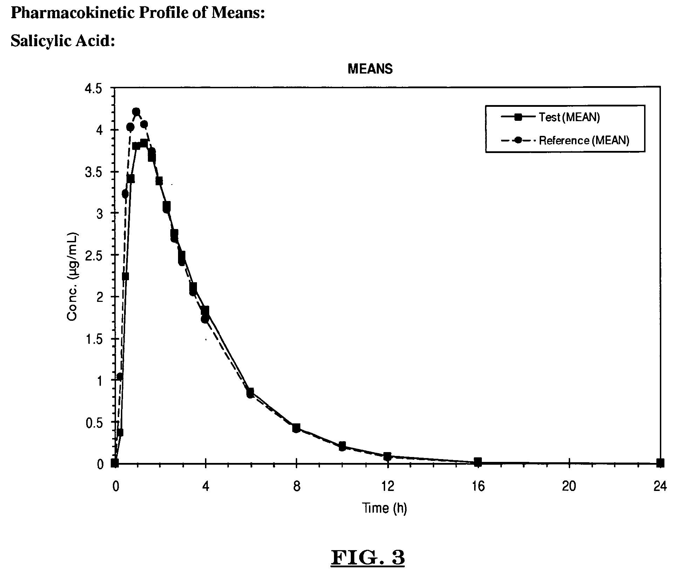

To monitor the in vitro and in vivo properties of the coated tablets, of the composition of the invention and conventional bisoprolol tablets the tablets were first tested in vitro and the results are plotted in Fig. 1. Tests for absorption in vivo were also carried out and the results are plotted in Figs. 2 and 3.

Further in vivo data was obtained for the combination product of Example 1. This data demonstrated that a time lag was sustained in the presence of aspirin. The aspirin absorption was much more rapid as reflected by the peak plasma level for aspirin which occurred approximately at ½ hour to one hour after administration.

Any possibility of adverse interaction in vivo arising on concomitant release of both drugs in the gastrointestinal tract is avoided. Thus, the release/dissolution and absorption of aspirin occurs first and to a substantial extent before the release and absorption of bisoprolol thereby reducing the likehood of in vivo adverse effects or patient injury.

Further, in medical practice, the composition of the invention has the considerable advantage of providing a single dose pharmaceutical product which combines anti-platelet action and antihypertensive action. In most cases only a single capsule is required per day. As a single product only is required the product is particularly advantageous from the point of view of compliance.

Formulation of a Bisoprolol Acetylsalicylic acid capsule

Samples of Bisoprolol aspirin capsules were manufactured by encapsulating uncoated acetylsalicylic acid tablets and the blend of bisoprolol fumarate with inert excipients. The stability of these capsules was evaluated by determining the level of impurities present in stored samples.

Any potential degradation of the product was determined by the quantification of (i) known degradation impurities, salicylic acid in the case of acetylsalicylic acid, and (RS)-1-(4-Hydroxymethylphenoxy)-3-isopropylaminopropan-2-ol (Impurity A) and 2-Isopropoxyethyl 4-[[(2RS)-2-Hydroxy-3-(isopropylamino)propyl}oxy}benzoate (Impurity K) in the case of Bisoprolol and (ii) any unknown impurities.

In the determination of impurities sample preparation was carried out as follows:

### *Sample Preparation*

Weigh accurately ten capsules and record the weight. Gently open the capsules and quantitatively transfer the contents to a 50 mL conical flask. Inspect each capsule shell to ensure all the contents have been transferred to the flask.

Using clean forceps separate the aspirin tablets from the flask.

Gently crack the coating of the tablet and transfer quantitatively to another 50 mL conical flask. Transfer by pipette 25.0 mL of diluent into each of the sample flasks.

Stopper the flasks and sonicate for 2 minutes with occasional swirling or until the content is completely disintegrated. Make sure there is no visible clumping of sample in the flasks.

Transfer the empty capsule shells to the bisoprolol sample flask and re-stopper flask.

Shake both flasks vigorously for 5 minutes.

Filter all sample solutions through a $0.45\mu$m filter, discarding the first 5-7ml, then carrying out a 1 in 50 dilution for the aspirin preparation and a 1 in 5 dilution for the bisoprolol preparation. Transfer sample to a HPLC vial for injection.

### *HPLC Analysis*

Samples were analysed by High Performance Liquid chromatography using UV detection and the following chromatographic conditions

Column:     Fortis 3mm, 150 mm x 3.0 mm i.d.

|  |  | Potassium phosphate buffer | Acetonitrile |
|---|---|---|---|
|  | 0 min | 85 % | 15 % |
| Mobile Phase gradient: | 15 min | 30 % | 70 % |
|  | 18 min | 70 % | 30 % |
|  | 21 min | 85 % | 15 % |

| Injection volume: | 10 μL |
| Flow rate: | 0.5 mL/min |
| Column Temperature: | 30 °C |
| Sample Temperature: | 4 °C |
| Needle Wash: | 50:50 acetonitrile:methanol |
| Detection: | UV |
| Wavelength: | 225 nm |

The quantity of any impurity present in either of the test solutions is calculated by comparing the area under the curve of any impurity peak to that of a standard active peak using external calibration as per equation 1. Impurity concentrations are expressed as %w/w. In the case of known impurities the relative response factor for that impurity is applied. In the case of unknown impurities the response (peak area) is compared to that of the active at a level of 0.1% in case of Bisoprolol and 0.15% in case of aspirin. (Ref Interpretation of chromatograms USP 26 p 2134). Any peak in the chromatogram at a concentration of <0.05% with respect to the active is disregarded in line with ICH guideline.

## Equation 1

$$\text{Analyte content} = \frac{A \times s \times P \times RRF \ (\% \ w/w)}{A' \times w}$$

A = response of analyte peak in the test item solution
A' = response of analyte peak in the calibration solution
s = concentration of analyte in calibration solution (mg/ml)
w = concentration of test item in test item solution (mg/ml)
P = purity of analyte standard (% w/w)
RRF = relative response factor

In TABLE 1 NMT means not more than.

## TABLE 1 - Results

| Impurity profile of Bisoprolol aspirin capsules with coated and uncoated tablets | | | | | | | | | | |
|---|---|---|---|---|---|---|---|---|---|---|
| **Storage: 25°C 60 % RH** | | | | | | | | | | |
| **Bisoprolol Acetylsalicylic acid 10/100mg Capsules (coated tablet)** | | | | | | | | | **Bisoprolol Acetylsalicylic acid 10/100mg Capsules (uncoated tablet)** | |
| | Specification | 0 Month | 3 Months | 6 Months | 12 Months | 18 Months | 24 Months | | 0 Months | 1 month |
| **Related Impurities - Acetylsalicylic acid** | | | | | | | | | | |
| Salicylic acid % | NMT 3.0% | 0.08 | 0.15 | 0.14 | 0.14 | 0.13 | 0.26 | | 0.04 | 0.24 |
| Largest Individual other Acetylsalicylic acid Impurity % | NMT 0.2% | < 0.05 | < 0.05 | 0.05 | < 0.05 | < 0.05 | < 0.05 | | <0.05 | <0.05 |
| Total Acetylsalicylic acid Impurities % | NMT 4.0% | 0.08 | 0.15 | 0.14 | 0.14 | 0.13 | 0.26 | | 0.04 | 0.24 |
| **Related Impurities - Bisoprolol** | | | | | | | | | | |
| Impurity A % | NMT 0.3% | < 0.05 | 0.05 | 0.05 | 0.05 | 0.05 | 0.05 | | <0.05 | <0.05 |
| Impurity K % | NMT 0.2% | < 0.05 | <0.05 | < 0.05 | < 0.05 | < 0.05 | < 0.05 | | <0.05 | <0.05 |
| Largest other individual unknown impurity % | NMT 0.2% | < 0.05 | <0.05 | < 0.05 | < 0.05 | 0.06 | < 0.05 | | 0.06 | 1.21 |
| Total Bisoprolol Impurities % | NMT 1.0% | < 0.05 | 0.05 | 0.06 | 0.05 | 0.11 | 0.05 | | 0.06 | 1.84 |

EP 2 857 014 B1

6

Acetylsalicylic acid is a weak acid, soluble in water. The stability of aspirin has been extensively studied and its sentitivity to moisture widely documented. Lewis J. Leeson and Albert M. Mattocks (J.Am.Pharm.Ass., Sci.Ed., 1958, 47, 329) have studied the stability of acetylsalicylic acid in the solid state. They conclude that the hydrolytic degradation of acetylsalicylic acid proceeds by a solution reaction mechanism in a mono-molecular layer of water on the acetylsalicylic acid crystal faces. Salicylic acid is the product of such degradation and its formation is a measure of the stability of the product.

Referring to Table 1, in capsules manufactured with uncoated tablets the increase in salicylic acid is evident following one months storage with a level of 0.24%. In the invention, the application of the coating results in a protection of the aspirin core against the ingress of moisture with a much slower increase in the level of salicylic acid. Following eighteen months storage at 25°C the level of salicylic acid is 0.13% with a level of 0.26% after 24 months at 25°C. The core is protected however, the coating does not hinder the release of aspirin in biological fluids.

Following storage for one month the Bisoprolol impurities detected in the product without coating indicate that the interaction of the two active components result in an unacceptable level of unknown degradation products, outside the guidelines of the International Committee on Harmonisation (ICH). Following one months storage at 25°C there is no increase in the levels of the two known impurities (RS)-1-(4-Hydroxymethylphenoxy)-3-isopropylaminopropan-2-ol (Impurity A) and 2-Isopropoxyethyl 4-[[(2RS)-2-Hydroxy-3-(isopropylamino)propyl}oxy}benzoate (Impurity K). However unknown impurities due to the interaction of the two active components are evident at a level of total impurities 1.84% and the largest of these unknown impurities is present at a level of 1.21%, six times the maximum allowable level for an unknown impurity according the ICH. This largest interaction impurity is detected in the chromatogram at approximately 9.5mins. Other potential interaction impurities that can occur are recorded in the chromatogram at approximately, 9.7, 13.5 and 15.2 mins. The inclusion of the coating layer on the tablet improves the stability of the product, with no evidence of such interaction products forming. Following twenty four months storage at 25°C there is no increase evident in the level of 'interaction impurities' with the total impurities present remaining consistent with initial levels of <0.05%. Thus, the capsule of the invention has a shelf life of at least 24 months.

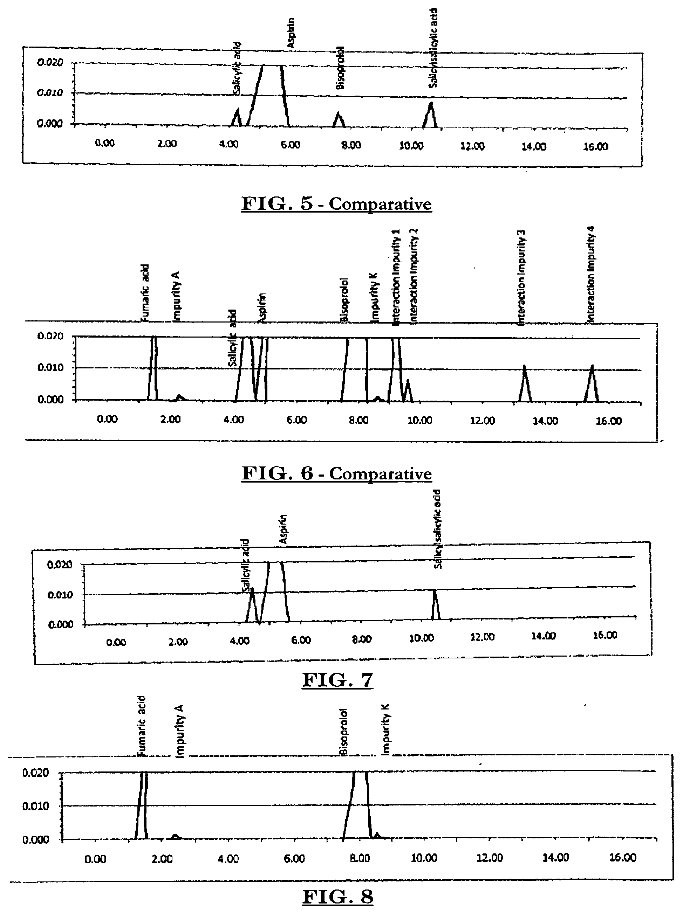

Referring to Figs. 5 to 8, in all cases the capsule contained 10mg bisoprolol fumarate and 100mg aspirin. In the case of a capsule containing an uncoated aspirin tablet and granules of bisoprolol fumarate the presence of several impurities are evident.

Fig. 5 represents a typical chromatogram from the analysis of the aspirin sample in capsules manufactured with uncoated aspirin tablets. Peaks are evident for aspirin, salicylic acid, salicylsalicylic acid and bisoprolol.

A typical chromatogram from the analysis of the aspirin from a capsule according to the invention is presented in Fig. 7. Peaks are evident for Aspirin, salicylic acid and salicylsalicylic acid. Salicylsalicylic acid is a synthetic impurity typically present in aspirin preparations at a level below 0.2%. Salicylic acid is both a synthetic impurity and a degradation product of aspirin. In contrast to Fig. 5, no bisoprolol impurity is evident in Fig. 7.

Fig. 6 represents a typical chromatogram from the analysis of the bisoprolol sample in capsules manufactured with uncoated tablets. Peaks are evident for fumaric acid, salicylic acid, aspirin, bisoprolol, impurity A, impurity K, and four unknown impurities at approximately 9.5, 9.7, 13.5 and 15.2 minutes resulting from the interaction of the aspirin and Bisoprolol.

A typical chromatogram from the analysis of the bisoprolol from a capsule according to the invention is presented in Fig 8 with peaks evident for fumaric acid and Bisoprolol. Bisoprolol is present in pharmaceutical preparations as the salt Bisoprolol fumarate and in solution the salt dissociates into bisoprolol and fumaric acid, resulting in two peaks in the chromatogram.

Summary Statements

1. A pharmaceutical capsule which contains only two pharmaceutically active ingredients, the capsule containing:-

a tablet which comprises aspirin as the first pharmaceutically active ingredient; and

granules surrounding the tablet, the granules comprising bisoprolol fumarate as the second pharmaceutically active ingredient,

the capsule containing from 50mg to 125mg of aspirin and from 1.25mg to 15mg of bisoprolol fumarate,

the aspirin tablet being coated with a barrier layer to prevent chemical interaction between the aspirin and the bisoprolol fumarate and to prevent interaction between aspirin and moisture in the capsule,

the aspirin being releasable from the capsule in biological fluids to provide a peak concentration in advance of a peak concentration of bisoprolol.

2. A pharmaceutical capsule of 1 wherein the aspirin is releasable to provide a peak concentration within about 2 hours.

3. A pharmaceutical capsule of 1 or 2 wherein the peak concentration of bisoprolol is provided within about 4 hours.

4. A pharmaceutical capsule formulation of any of 1 to 3 wherein, when the capsule is stored at 25°C and 60% relative humidity for 1 month, the concentration of any single impurity is not greater than 0.3% w/w.

5. A pharmaceutical capsule formulation of 4 wherein the capsule is stored at 25°C and 60% relative humidity for 3 months, or for 6 months, or for 12 months, or for up to 24 months, the concentration of any single impurity is not greater than 0.3% w/w.

6. A pharmaceutical capsule formulation of any of 1 to 5 wherein when the capsule is stored at 25°C and 60% relative humidity for 1 month the concentration of any single impurity is not greater than 0.2% w/w.

7. A pharmaceutical capsule formulation of 6 wherein when the capsule is stored at 25°C and 60% relative humidity for 3 months, or for 6 months, or for 12 months, or for up to 24 months, the concentration of any single impurity is not greater than 0.2% w/w.

8. A pharmaceutical capsule of any of 1 to 7 wherein the tablet contains from 75 to 110mg of aspirin.

9. A capsule of any of 1 to 8 wherein the tablet contains approximately 75mg of aspirin.

10. A capsule of any of 1 to 8 wherein the tablet contains approximately 100mg of aspirin.

11. A capsule of any of 1 to 8 wherein the tablet contains approximately 82mg of aspirin.

12. A capsule of any of 1 to 11 wherein the granules contain approximately 5mg of bisoprolol fumarate.

13. A capsule of any of 1 to 11 wherein the granules contain approximately 10mg of bisoprolol fumarate.

14. A capsule of any of 1 to 11 wherein the granules contain approximately 1.25mg of bisoprolol fumarate.

15. A capsule of any of 1 to 11 wherein the granules contain approximately 3.75mg of bisoprolol fumarate.

16. A capsule of any of 1 to 11 wherein the granules contain approximately 2.5mg of bisoprolol fumarate.

The invention is not limited to the embodiment hereinbefore described which may be varied in detail.

## Claims

1. A pharmaceutical capsule which contains only two pharmaceutically active ingredients, the capsule containing:-

    a tablet which comprises aspirin as the first pharmaceutically active ingredient; and
    bisoprolol fumarate with a particle size controlled to 90% < 425 $\mu$m, as the second pharmaceutically active ingredient, blended with inert excipients and surrounding the tablet;
    the capsule containing from 50 mg to 125 mg of aspirin and from 1.25 mg to 15 mg of bisoprolol fumarate;
    the aspirin tablet being coated with a barrier layer to prevent chemical interaction between the aspirin and the bisoprolol fumarate and to prevent interaction between aspirin and moisture in the capsule; and
    the aspirin being releasable from the capsule in biological fluids to provide a peak concentration in advance of a peak concentration of bisoprolol.

2. A pharmaceutical capsule as claimed in claim 1 wherein the aspirin is for immediate release from the capsule so as to provide the peak concentration of aspirin within about 2 hours.

3. A pharmaceutical capsule as claimed in claim 1 or 2 wherein the bisoprolol is for immediate release from the capsule so as to provide the peak concentration of bisoprolol within about 4 hours.

4. A pharmaceutical capsule formulation as claimed in any of claims 1 to 3 wherein, when the capsule is stored at 25°C and 60% relative humidity for 1 month, the concentration of any single impurity is not greater than 0.3% w/w.

5. A pharmaceutical capsule formulation as claimed in claim 4 wherein the capsule is stored at 25°C and 60% relative humidity for 3 months, or for 6 months, or for 12 months, or for up to 24 months, the concentration of any single impurity is not greater than 0.3% w/w.

6. A pharmaceutical capsule formulation as claimed in any of claims 1 to 5 wherein when the capsule is stored at 25°C and 60% relative humidity for 1 month the concentration of any single impurity is not greater than 0.2% w/w.

7. A pharmaceutical capsule formulation as claimed in claim 6 wherein when the capsule is stored at 25°C and 60% relative humidity for 3 months, or for 6 months, or for 12 months, or for up to 24 months, the concentration of any single impurity is not greater than 0.2% w/w.

8. A pharmaceutical capsule as claimed in any of claims 1 to 7 wherein the tablet contains from 75 to 110 mg of aspirin.

9. A capsule as claimed in any of claims 1 to 8 wherein the tablet contains approximately 75 mg of aspirin.

10. A capsule as claimed in any of claims 1 to 8 wherein the tablet contains approximately 100 mg of aspirin.

11. A capsule as claimed in any of claims 1 to 8 wherein the tablet contains approximately 82 mg of aspirin.

12. A capsule as claimed in any of claims 1 to 11 wherein the capsule contains approximately 5 mg of bisoprolol fumarate.

13. A capsule as claimed in any of claims 1 to 11 wherein the capsule contains approximately 10 mg of bisoprolol fumarate.

14. A capsule as claimed in any of claims 1 to 11 wherein the capsule contains approximately 1.25 mg of bisoprolol fumarate.

15. A capsule as claimed in any of claims 1 to 11 wherein the capsule contains approximately 3.75 mg of bisoprolol fumarate.

16. A capsule as claimed in any of claims 1 to 11 wherein the capsule contains approximately 2.5 mg of bisoprolol fumarate.

**Patentansprüche**

1. Pharmazeutische Kapsel, die nur zwei pharmazeutische Wirkstoffe enthält, wobei die Kapsel Folgendes enthält:

   eine Tablette, die Aspirin als ersten pharmazeutischen Wirkstoff umfasst und
   Bisoprololfumarat mit einer Partikelgröße, die auf 90 % <425 $\mu$m geregelt ist, als zweiten pharmazeutischen Wirkstoff umfasst, das mit inerten Hilfsstoffen vermischt ist und die Tablette umgibt;
   wobei die Kapsel von 50 mg bis 125 mg Aspirin und von 1,25 mg bis 15 mg Bisoprololfumarat enthält,
   wobei die Aspirin-Tablette mit einer Barriereschicht beschichtet ist, um eine chemische Wechselwirkung zwischen dem Aspirin und dem Bisoprololfumarat zu verhindern und um eine Wechselwirkung zwischen Aspirin und Feuchtigkeit in der Kapsel zu verhindern, und
   wobei das Aspirin aus der Kapsel in biologische Flüssigkeiten freisetzbar ist, um eine Höchstkonzentration im Voraus einer Höchstkonzentration von Bisoprolol bereitzustellen.

2. Pharmazeutische Kapsel nach Anspruch 1, worin das Aspirin aus der Kapsel sofort freisetzbar ist, um die Höchstkonzentration von Aspirin innerhalb von etwa 2 Stunden bereitzustellen.

3. Pharmazeutische Kapsel nach Anspruch 1 oder 2, worin das Bisoprolol aus der Kapsel sofort freisetzbar ist, um die Höchstkonzentration von Bisoprolol innerhalb von etwa 4 Stunden bereitzustellen.

**4.** Pharmazeutische Kapselformulierung nach einem der Ansprüche 1 bis 3, worin, bei Aufbewahrung der Kapsel bei 25°C und 60 % relativer Feuchte für 1 Monat, die Konzentration von jedweder einzelnen Verunreinigung nicht größer als 0,3 % m/m ist.

**5.** Pharmazeutische Kapselformulierung nach Anspruch 4, worin, bei Aufbewahrung der Kapsel bei 25°C und 60 % relativer Feuchte für 3 Monate oder für 6 Monate oder für 12 Monate oder für bis zu 24 Monate, die Konzentration von jedweder einzelnen Verunreinigung nicht größer als 0,3 % m/m ist.

**6.** Pharmazeutische Kapselformulierung nach einem der Ansprüche 1 bis 5, worin, bei Aufbewahrung der Kapsel bei 25°C und 60 % relativer Feuchte für 1 Monat, die Konzentration von jedweder einzelnen Verunreinigung nicht größer als 0,2 % m/m ist.

**7.** Pharmazeutische Kapselformulierung nach Anspruch 6, worin, bei Aufbewahrung der Kapsel bei 25°C und 60 % relativer Feuchte für 3 Monate oder für 6 Monate oder für 12 Monate oder für bis zu 24 Monate, die Konzentration von jedweder einzelnen Verunreinigung nicht größer als 0,2 % m/m ist.

**8.** Pharmazeutische Kapsel nach einem der Ansprüche 1 bis 7, worin die Tablette von 75 bis 110 mg Aspirin enthält.

**9.** Kapsel nach einem der Ansprüche 1 bis 8, worin die Tablette ungefähr 75 mg Aspirin enthält.

**10.** Kapsel nach einem der Ansprüche 1 bis 8, worin die Tablette ungefähr 100 mg Aspirin enthält.

**11.** Kapsel nach einem der Ansprüche 1 bis 8, worin die Tablette ungefähr 82 mg Aspirin enthält.

**12.** Kapsel nach einem der Ansprüche 1 bis 11, worin die Kapsel ungefähr 5 mg Bisoprololfumarat enthält.

**13.** Kapsel nach einem der Ansprüche 1 bis 11, worin die Kapsel ungefähr 10 mg Bisoprololfumarat enthält.

**14.** Kapsel nach einem der Ansprüche 1 bis 11, worin die Kapsel ungefähr 1,25 mg Bisoprololfumarat enthält.

**15.** Kapsel nach einem der Ansprüche 1 bis 11, worin die Kapsel ungefähr 3,75 mg Bisoprololfumarat enthält.

**16.** Kapsel nach einem der Ansprüche 1 bis 11, worin die Kapsel ungefähr 2,5 mg Bisoprololfumarat enthält.

**Revendications**

**1.** Capsule pharmaceutique ne contenant que deux ingrédients pharmaceutiquement actifs, la capsule contenant :

un comprimé qui comprend de l'aspirine comme premier ingrédient pharmaceutiquement actif ; et
du fumarate de bisoprolol ayant une taille de particules contrôlée à 90% < 425 $\mu$m comme deuxième ingrédient pharmaceutiquement actif, mélangé avec des excipients inertes et entourant le comprimé ;
la capsule contenant de 50 mg à 125 mg d'aspirine et de 1,25 mg à 15 mg de fumarate de bisoprolol ;
le comprimé d'aspirine étant revêtu d'une couche barrière afin d'éviter une interaction chimique entre l'aspirine et le fumarate de bisoprolol, et d'éviter une interaction entre l'aspirine et l'humidité dans la capsule ; et
l'aspirine pouvant être libérée à partir de la capsule dans des fluides biologiques afin de fournir une concentration maximale en avance par rapport à une concentration maximale de bisoprolol.

**2.** Capsule pharmaceutique selon la revendication 1, dans laquelle l'aspirine est formulée pour être libérée immédiatement à partir de la capsule de manière à ce que la concentration maximale en aspirine soit atteinte en environ 2 heures.

**3.** Capsule pharmaceutique selon la revendication 1 ou 2, dans laquelle le bisoprolol est formulé pour être libéré immédiatement à partir de la capsule de manière à ce que la concentration maximale en bisoprolol soit atteinte en environ 4 heures.

**4.** Formulation de capsule pharmaceutique selon l'une quelconque des revendications 1 à 3, dans laquelle, lorsque la capsule est stockée à 25°C et 60% d'humidité relative pendant 1 mois, la concentration en une impureté unique quelconque n'est pas supérieure à 0,3% p/p.

5. Formulation de capsule pharmaceutique selon la revendication 4, dans laquelle, lorsque la capsule est stockée à 25°C et 60% d'humidité relative pendant 3 mois, ou pendant 6 mois, ou pendant 12 mois, ou jusqu'à 24 mois, la concentration en une impureté unique quelconque n'est pas supérieure à 0,3% p/p.

6. Formulation de capsule pharmaceutique selon l'une quelconque des revendications 1 à 5, dans laquelle, lorsque la capsule est stockée à 25°C et 60% d'humidité relative pendant 1 mois, la concentration en une impureté unique quelconque n'est pas supérieure à 0,2% p/p.

7. Formulation de capsule pharmaceutique selon la revendication 6, dans laquelle, lorsque la capsule est stockée à 25°C et 60% d'humidité relative pendant 3 mois, ou pendant 6 mois, ou pendant 12 mois, ou jusqu'à 24 mois, la concentration en une impureté unique quelconque n'est pas supérieure à 0,2% p/p.

8. Capsule pharmaceutique selon l'une quelconque des revendications 1 à 7, dans laquelle le comprimé contient de 75 à 110 mg d'aspirine.

9. Capsule selon l'une quelconque des revendications 1 à 8, dans laquelle le comprimé contient environ 75 mg d'aspirine.

10. Capsule selon l'une quelconque des revendications 1 à 8, dans laquelle le comprimé contient environ 100 mg d'aspirine.

11. Capsule selon l'une quelconque des revendications 1 à 8, dans laquelle le comprimé contient environ 82 mg d'aspirine.

12. Capsule selon l'une quelconque des revendications 1 à 11, dans laquelle la capsule contient environ 5 mg de fumarate de bisoprolol.

13. Capsule selon l'une quelconque des revendications 1 à 11, dans laquelle la capsule contient environ 10 mg de fumarate de bisoprolol.

14. Capsule selon l'une quelconque des revendications 1 à 11, dans laquelle la capsule contient environ 1,25 mg de fumarate de bisoprolol.

15. Capsule selon l'une quelconque des revendications 1 à 11, dans laquelle la capsule contient environ 3,75 mg de fumarate de bisoprolol.

16. Capsule selon l'une quelconque des revendications 1 à 11, dans laquelle la capsule contient environ 2,5 mg de fumarate de bisoprolol.

**FIG. 1**

Pharmacokinetic Profile of Means:
Aspirin

**FIG. 2**

**Pharmacokinetic Profile of Means:**

**Salicylic Acid:**

FIG. 3

**Pharmacokinetic Profile of Means:**

**Bisoprolol:**

FIG. 4

EP 2 857 014 B1

**FIG. 5 - Comparative**

**FIG. 6 - Comparative**

**FIG. 7**

**FIG. 8**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LEWIS J. LEESON ; ALBERT M. MATTOCKS.** *J.Am.Pharm.Ass., Sci.Ed.,* 1958, vol. 47, 329